# EUROPEAN PATENT SPECIFICATION

(11) **EP 1 933 707 B1**
(45) Date of publication and mention of the grant of the patent: **02.01.2019**
(21) Application number: 06825636.1
(22) Date of filing: 09.10.2006
(51) Int. Cl.: A61B 6/00, G01N 33/543

(54) **METHOD FOR MONITORING A CHILDBIRTH PROCESS**
VERFAHREN ZUR ÜBERWACHUNG VON GEBURTSPROZESSEN
PROCEDE DE SURVEILLANCE D'UN PROCESSUS D'ACCOUCHEMENT

(30) Priority: 12.10.2005 US 596673 P
(43) Date of publication of application: 25.06.2008
(73) Proprietor: ObsteCare AB, 171 65 Solna (SE)
(72) Inventor: ITZEL, Johan, S-182 39 Danderyd (SE); WIBERG-ITZEL, Eva, S-182 39 Danderyd (SE)
(74) Representative: Brann AB
(86) International application number: PCT/US2006/039377
(87) International publication number: WO 2007/047196

(56) References cited:
- WO-A1-2005/034762
- WO-A2-2004/097365
- US-A1- 2002 060 247
- US-A1- 2003 171 666
- US-A1- 2004 254 121
- ITZEL ET AL: "The association between lactate in vaginal fluid ('LAC-test') and time to spontaneous onset of labour in cases with suspected PPROM", AMERICAN JOURNAL OF OBSTETRICS & GYNECOLOGY, MOSBY, ST LOUIS, MO, US, vol. 193, no. 6, 1 December 2005 (2005-12-01), page S6, XP005405731, ISSN: 0002-9378, DOI: 10.1016/J.AJOG.2005.10.206
- EVA WIBERG-ITZEL ET AL: "Lactate Determination in Vaginal Fluids: a new method in the diagnosis of prelabour rupture of membranes", BJOG: AN INTERNATIONAL JOURNAL OF OBSTETRICS AND GYNAECOLOGY, WILEY-BLACKWELL PUBLISHING LTD, GB, vol. 112, no. 6, 1 June 2005 (2005-06-01), pages 754-758, XP002506981, ISSN: 1470-0328, DOI: 10.1111/J.1471-0528.2004.00521.X [retrieved on 2004-12-17]
- E WIBERG-ITZEL ET AL: "Association between lactate in vaginal fluid and time to spontaneous onset of labour for women with suspected prelabour rupture of the membranes", BJOG: AN INTERNATIONAL JOURNAL OF OBSTETRICS AND GYNAECOLOGY, vol. 113, no. 12, 29 September 2006 (2006-09-29), - 27 December 2006 (2006-12-27), pages 1426-1430, XP055195545, ISSN: 1470-0328, DOI: 10.1111/j.1471-0528.2006.01088.x
- KRUGER ET AL: "Predictive value of fetal scalp blood lactate concentration and pH as markers of neurologic disability", AMERICAN JOURNAL OF OBSTETRICS & GYNECOLOGY, MOSBY, ST LOUIS, MO, US, vol. 181, no. 5, 1 November 1999 (1999-11-01), pages 1072-1078, XP005691107, ISSN: 0002-9378, DOI: 10.1016/S0002-9378(99)70083-9
- NORDSTRÖM L: "Fetal scalp and cord blood lactate", BEST PRACTICE & RESEARCH CLINICAL OBSTETRICS AND GYNAECOLOGY,, vol. 18, no. 3, 1 January 2004 (2004-01-01), pages 467-476, XP008108927,
- DAVID B. PYNE ET AL: "Evaluation of the Lactate Pro blood lactate analyser", EUROPEAN JOURNAL OF APPLIED PHYSIOLOGY, vol. 82, no. 1-2, 15 May 2000 (2000-05-15) , pages 112-116, XP055337756, DE ISSN: 1439-6319, DOI: 10.1007/s004210050659
- CRAIG E. PENNELL ET AL: "A New Method for Rapid Measurement of Lactate in Fetal and Neonatal Blood", AUSTRALIAN AND NEW ZEALAND JOURNAL OF OBSTETRICS & GYNAECOLOGY., vol. 39, no. 2, 1 May 1999 (1999-05-01), pages 227-233, XP055337820, AU ISSN: 0004-8666, DOI: 10.1111/j.1479-828X.1999.tb03379.x

## Description

### Technical Field

The present invention relates to a method for monitoring a childbirth process of a pregnant woman.

### Background of the Invention

One problem in today's delivery methods is that women suffer from dystocya during labor. This could result in that the delivery does not progress as desired and that the labor is drawn out without a successful natural childbirth. The pregnant woman may become frustrated and it may be necessary to use methods such as, vacuum, forceps or caesarean to deliver the baby. The dystocya of the pregnant woman may also expose the fetus to injury and fatigue.

The lactate concentration in the blood of the fetus has been measured in the past to control that the fetus does not suffer from oxygen deficiency. However, the lactate concentration in the fetus does not indicate the condition of the pregnant woman. There is a need to more effectively determine and control the condition of woman suffering from dystocya at an early stage to avoid unnecessary labor before using surgical and alternative childbirth methods. There is also a need for an effective apparatus to be able to determine the above-outlined conditions. In addition to the above needs, the apparatus should also handle at least cervix dilatation data, fetal positioning data and fetal lactate and pH measurements WO 2005/034762 relates to a method of monitoring childbirth process of pregnant woman, involves subjecting pregnant women to alternative childbirth options, when lactate concentration in vaginal fluid, is greater than threshold level, and discloses a method for monitoring a childbirth process of a pregnant woman: in a first measuring step, a first lactate concentration of vaginal fluids is measured; in a comparison step, it is determined if the measured lactate concentration is greater than a predetermined lactate concentration value that indicates that amniotic fluid has passed from amnion of the pregnant woman and the membrane has ruptured.

### Summary of the Invention

The method of the present invention provides a solution to the above-outlined problems. More particularly, the method is for tagging disposable articles that are used together with an apparatus for monitoring a childbirth process of a pregnant woman. The use of an identification mechanism such as a tag makes sure that the liquid carrying elements that are used together with the apparatus have the correct production origin. The monitoring of the childbirth process is done by an apparatus that measures lactate concentrations and evaluates the levels to determine whether the lactate concentrations are changing. The apparatus may, among other things measure, enter and display the information from the respective methods and sensors.

Together with the apparatus a multitude of liquid carrying disposables may be used. In order to ensure that the measurements presented to the medical practitioner is correct it is important that the correct disposables are used in order to not give inaccurate information. The disposables of the present invention may therefor be marked with a tag or any other suitable identification method that can be detected by the apparatus.

### Brief Description of the Drawing

Fig. 1 is a schematic flow chart showing some of the steps of the method of the present invention;
Fig. 2 is a schematic illustration of graphical representation of measured data;
Fig. 3 is a schematic illustration of a test strip and a measurement device;
Fig. 4 is an exploded perspective view of an alternative embodiment of the device of the present invention;
Fig. 5 is a perspective view of the device shown in Fig. 4; and
Fig. 6 is a perspective view of the device inserted in a display unit.

### Detailed Description

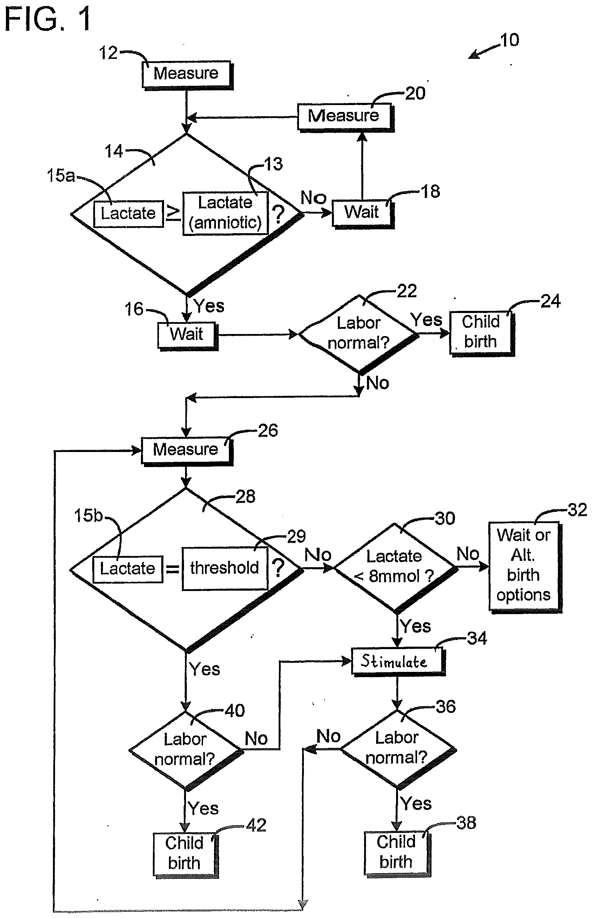

With reference to Fig. 1, the method 10 of the present invention includes a measuring step 12 that measures a lactate concentration 15a in fluids, such as vaginal fluids, in connection with pregnancy to determine whether the amniotic fluids have passed or are in the process of being passed from the amnion. In general, the uterus muscle of pregnant women produces lactate so that the lactate concentration of the vaginal fluids may be measured to provide a measurement of the amount of lactate produced by the uterus muscle. Non-pregnant women often have no or very little lactate in the vaginal fluids.

If the lactate concentration 15a is higher than a predetermined lactate concentration 13, such as 4-5 mmol/l, more preferably higher than 4.5 mmol/l, as indicated in a comparison step 14 then it may be concluded that the membrane has ruptured and amniotic fluids likely have passed and that the childbirth labor is likely to start after a waiting period 16. It is to be understood that the 4-5 mmol/l is an illustrative example that applies to most women and that the invention is not limited to the values used in the examples.

If the lactate concentration is lower than 4.5 mmol/l then there is a high likelihood that the amniotic fluids are still contained within the amnion. The lactate concentration may again be measured in a measuring step 20 after a waiting period 18. It is again determined in the comparison step 14 whether the lactate concentration is more or less than 4.5 mmol/l. If the lactate concentration is again below 4.5 mmol/l, a second measuring may be conducted later and the measuring may be repeated at suitable time intervals until the lactate concentration exceeds 4.5 mmol/l or it is obvious that the amniotic fluids have passed.

As indicated above, if the lactate concentration measured in the measuring step 12 is above 4.5 mmol/l, the next step is to wait for about two days or so to see if the woman starts the labor by herself. In a determining step 22, it is determined whether the labor has started or not. If the labor has started and is progressing normally then the childbirth procedure 24 may proceed. If it is determined in the determining step 22 that the labor has not started or the labor is not progressing normally, a lactate concentration 15b is measured in a measuring step 26.

In a comparison step 28 it is then determined if the lactate concentration 15b as measured in the measuring step 26 is within a lactate threshold interval 29 that may be about 8-10 mmol/l. If the lactate concentration as measured in the step 26 is not within the threshold interval 29, then it is determined in a comparison step 30 whether the lactate concentration is less than the threshold interval 29 or about 8 mmol/l. If the lactate concentration as measured in step 26 is greater than the threshold interval 29 then a waiting step 32, such as a couple of hours, may start to see if the labor progress normally. If labor does not progress normally, alternative childbirth options may be considered such as caesarean, forceps or the use of suction cups that are connected to vacuum to draw out the baby. An important feature of the present invention is that the monitoring of the lactate concentration may be used to predict whether the woman is likely to give a natural birth or not without forcing the pregnant woman to go through long and agonizing efforts to give birth. It is therefore possible to use alternative childbirth options at a relatively early stage. It is to be understood that the 8-10 mmol/l is an illustrative example that applies to most women and that the invention is not limited to the values used in the examples.

If the lactate concentration, as measured in step 26, is less than the threshold interval 29, then the woman may be stimulated with drugs or other aids to give birth in a stimulation step 34. In a determining step 36, it may be determined if the labor is progressing normally. If the labor is progressing normally the woman may proceed to give birth 38. If the labor is not progressing normally, the lactate concentration may again be measured in the measuring step 26 and the process continues in the comparison step 28, as described above.

If it is determined in the comparison step 28 that the lactate concentration, as measured in step 26, is at the threshold interval 29, such as between 8-10 mmol/l, then it is determined whether the labor is progressing normally in a determining step 40. If labor is progressing normally, the woman may proceed to give birth 42. If labor is not progressing normally, the woman may be stimulated to give birth in the stimulation step 34 and the process continues to the determining step 36, as described above.

The various processing loops may continue until the woman either gives birth by herself or is subjected to alternative childbirth options. As indicated above, an important feature of the present invention is that the woman may be prevented from agonizing and long childbirth efforts before alternative childbirth options are used. Alternative childbirth options may be used at an earlier stage when the lactate concentration indicates that the uterus muscle is operating above the lactate threshold without resulting in a natural childbirth.

Another important feature of the present invention is to use a measuring and presentation device to effectively present the measured lactate values together with the time lapsed. The depicted time intervals could be both on a relative time (time from start) or absolute time (time of day) to indicate the trends of the measured lactate values. In addition, the device may also be used to measure lactate levels in the fetus such as in the scalp blood of the fetus. The device may also be used to present the how far down in the pelvis the head of the fetus has penetrated and the dilation of the cervix.

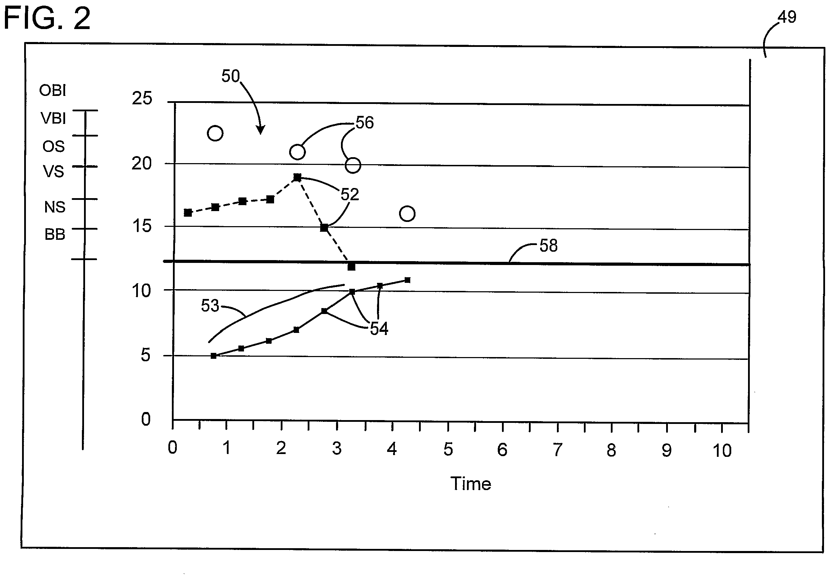

Fig. 2 is a display 50 is an illustrative example that shows lactate data 52 of the pregnant woman over time, lactate data 53 of the fetus, cervix dilation data 54 and fetal head positioning data 56. For example, the lactate data 52 shows an upward trend until the addition of stimulation substances to the pregnant woman is turned off and the lactate level is dramatically reduced. Because the display 50 shows that the lactate levels of the woman is already very high and rising, such as values above 15 mmol per liter, the medical practitioners may learn that there is no longer a need to provide stimulation to the pregnant woman. In this way, the display 50 may be used to prevent the misuse of stimulating substances. The medical practitioner may also use the trends indicated by the data 53, 54, 56 so that the practitioner can see all the data together and take all the information into account before making a decision about what action to take including medication required and the need for performing a caesarian or any other such aided child birth. For example, the cervix dilation data 54 may be compared to a normal cervix dilation curve 55 that applies to most women. The medical practitioner may also use the display 50 to see how the pregnant woman and fetus are reacting to treatment.

The practitioner may take a plurality of measurements of the lactate concentrations 52 at time intervals. The measured lactate concentrations 52 are then presented at the time intervals on the display 50. The lactate concentrations 52 are evaluated to determine whether the lactate concentrations 52 are rising to indicate that an individual lactate threshold value 58 of the pregnant woman has been exceeded. To be able to determine that the individual lactate threshold has been exceeded is particularly important for women who have an unusually high or low lactate threshold.

All the data 52, 53, 54, 56 and other data that have been displayed on the display 50 may be saved for later retrieval together with information of the time the data was measured. The data may be stored together with patient information. This data may be electronically transferred to remote equipment such as electronic medical records by using wireless or wired communication. The device may also be equipped with an automatic decision guiding and alarm system that is based on measured lactate values from mother, measured fetal lactate values, measured fetal pH values. The system may also base decisions and alarms on the opening size of cervix and the progression of the fetus head in the pelvis. Of course, a combination of the above information may be used. The devise 50 should be designed so that it can handle several childbirth processes at the same time in the same device. The stored data in the device may have the ability to enter and present the measured data in electronic medical records.

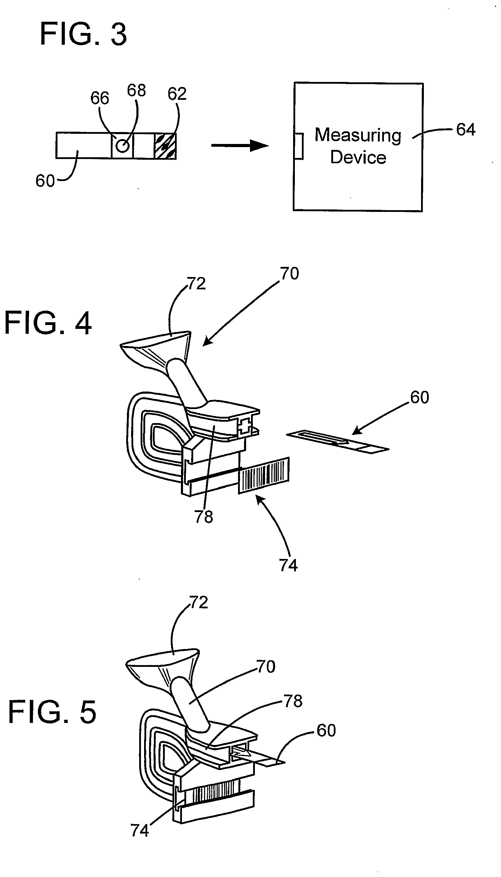

Fig. 3 shows a liquid carrying element such as a test strip 60 that is insertable into a measurement device 64. Fig. 3 is only an illustrative example of a liquid carrying element and the present invention is not limited to the test strip and the measurement device as shown. When a sample of liquid is collected for use with the measurement and presentation device the test strip or liquid carrying element 60 may be used to collect a liquid 62 from the pregnant woman that is to be analyzed. Preferably, the liquid to be analyzed is amniotic fluid or blood. The test strip may be inserted into a measurement and presentation device 64 either prior to or after the collection of the liquid. Preferably, the liquid 62 is deposited on the test strip 60. The device 64 then analyzes the liquid 62 such as determining a lactate concentration 15a, as described above. The test strips may be inserted in the measurement and presentation device as individual elements or as magazines containing several test strips. The measurement device 64 and the test strip 60 are both essential to make sure the data is accurate. The use of a non-conforming test strip can cause malfunction, or even worse, that invalid data is presented and, as a consequence, the pregnant woman is incorrectly treated.

Another important feature of the present invention is that in order to make sure that the correct test strip 60 is inserted in the measurement device 64 an identification mechanism 66 may be used on the test strip 60. The test strip may contain an identification tag 68. It is essential for correctness of the measured result that no contamination or other source of error is induced in the handling chain of the test sample of the liquid to be analyzed or measured. The tag 68 may therefore be designed so that the tag is detected by the measurement device 64 and give the measurement device the possibility of discriminating between correctly tagged test strips and untagged or unacceptable pirate strips. The tagging of the strips is a way to validate the origin of the test strips.

Also, other liquid carrying elements like tubes, catheters and bowls may be used in conjunction with the measurement device 64 and all such liquid carrying elements may also be tagged, as described above.

A wide variety of methods of tagging the strips or elements 60 may be used. For example, the tagging method may include a tuned antenna, resistive element on which the current/voltage is measured, mechanical key that can be detected, bar code that can be automatically scanned, radio frequency identification mechanisms or reflecting elements that can be optically read. The above mechanisms are only examples of how the mechanism of tagging in practice can be realized and the present invention of tagging the liquid carrying elements are not limited to the above examples.

The tag may apart from being used as a detection mechanism, also be used to validate the correct origin of the liquid carrying element and to carry other types of information. This information is supplementary and the absence of such does not mean that a tagging mechanism has not been implemented. The supplementary information that can be carried is for example, production date, calibration data, production batch information or any other suitable information.

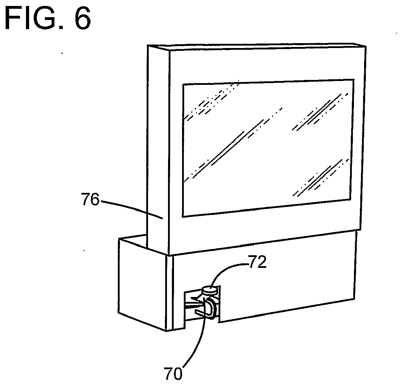

Figs. 4-6 are perspective views of an alternative embodiment of the device of the present invention. The test strip 60 may be inserted into an other liquid carrying element 70 that has an inlet 72 defined therein for receiving liquid to be tested such as amniotic fluid discussed above. Identification tag or bar code 74 may be attached to the element 70 for identification purposes. Fig. 5 shows the element 70 completely assembled. The element 70 may then be inserted into and removably attached to a measurement device 76. The element 70 is removed therefrom when the analysis of the information has been displayed as a result of the information on the strip 60. The element 70 has guiding members 78 to ensure the element 70 is correctly inserted into the device 76. Once the element 70 (including the strip 60 and identification tag 74) is attached to the device 76, the liquid is poured into the inlet 72 and the strip 60 measures the content of the liquid. When the analysis is complete, the entire element 70 is removed from the device 76 and may be thrown away. The element 70 may contain a filter to ensure that foreign substances are removed from the measured samples

## Claims

1. A method of monitoring a childbirth process of a pregnant woman, comprising:
• providing an amniotic fluid (62) sample obtained from the pregnant woman during labor;
• carrying the amniotic fluid (62) with a liquid carrying element (60, 70);
• filtering the amniotic fluid disposed in the liquid carrying element (60, 70) to avoid foreign substances; and
• determining *in vitro* a lactate concentration of the amniotic fluid (62) carried by the liquid carrying element (60, 70) by inserting the liquid carrying element (60, 70) into a lactate measuring device (64, 76),
wherein the lactate measuring device is capable of electronically transferring data to remote equipment, such as by wireless transfer,
to predict a progress of a childbirth delivery.

2. A method according to claim 1, wherein the method further comprises placing the amniotic fluid on a liquid carrying element in the form of a test strip (60).

3. A method according to claim 2, wherein the method further comprises inserting the test strip in a disposable casing (70).

4. A method according to any one of the preceding claims, wherein the method further comprises tagging the liquid carrying element and setting the lactate measuring device (64, 76) to detect tagged liquid carrying elements.

5. A method according to claim 1, or 4, wherein the lactate measuring device (64, 76) measures a liquid carried by a catheter.

6. A method according to any one of the preceding claims, wherein the method further comprises adding an identification mechanism on the liquid carrying element (60, 70).

7. A method according to claim 6, wherein the lactate measuring device (64, 76) detects an identification mechanism on the liquid carrying element (60, 70) prior to determining a lactate concentration of the amniotic fluid (62).

8. A method according to any one of the preceding claims, wherein the method further comprises tagging the liquid carrying element (60, 70) by providing a bar code.

9. A method according to claim 1 wherein the method further comprises adding measurement calibration data, production date, and/or production batch information to the liquid carrying element (60, 70).

10. A system for collecting and measuring *in vitro* lactate in amniotic fluid, which system comprises
- a lactate measuring device (64, 76) capable of electronically and wirelessly transferring data to remote equipment, and
- a liquid carrying element (60, 70), which is arranged for collecting an amniotic fluid sample (62) obtained from a pregnant woman during labor and which is insertable into said lactate measuring device (64, 76), wherein the liquid carrying element contains a filter for removing foreign substances.

11. The system according to claim 10, wherein the liquid carrying element is tagged and includes an identification mechanism (66) enabling detection by the measuring device of tagged liquid carrying element(s) and/or validation of the origin of the liquid carrying element.

12. The system according to claim 11, wherein the tagged liquid carrying element carries information regarding production date, calibration data, and/or production batch.

13. The system according to any one of claims 10-12, wherein the lactate measuring device is arranged for presenting measured lactate values together with time lapsed from obtaining the amniotic fluid, enabling a prediction of progress of a childbirth delivery.

14. The system according to any one of claims 10-13, wherein the lactate measuring device can generate alarms based on measured lactate values.

15. The system according to any one of claims 10-14, wherein the lactate measuring device can store patient information together with measured lactate values, optionally wherein the lactate measuring device can store information from multiple patients at the same time.

16. The system according to any one of claims 10-15, wherein the liquid carrying element is a test strip (60).

17. A system according claim 16, wherein the test strip has been inserted into a disposable liquid carrying element (70).

18. The system according to any one of claims 10-15, wherein the liquid carrying element (60, 70) is a catheter.

19. The system according to any one of claims 10-18, wherein the liquid carrying element (60,70) has been tagged by a bar code.

20. The system according to any one of claims 10-19, wherein the lactate measuring device (64, 76) is further arranged for the measurement of lactate levels of a fetus at or after the onset of labour, and wherein the liquid carrying element (60, 70) is further arranged to carry blood from said fetus, optionally wherein the blood is scalp blood.

## Patentansprüche

1. Verfahren zum Überwachen von Geburtsprozessen einer schwangeren Frau, umfassend:
• Bereitstellen einer Fruchtwasser(62)-Probe, die von der schwangeren Frau während der Wehen erhalten wurde;
• Tragen des Fruchtwassers (62) mit einem flüssigkeitstragenden Element (60, 70);
• Filtern des Fruchtwassers, das sich in dem flüssigkeitstragenden Element (60, 70) befindet, um Fremdstoffe zu vermeiden; und
• Bestimmen einer Lactatkonzentration des durch das flüssigkeitstragende Element (60, 70) getragenen Fruchtwassers (62) *in* vitro durch Einsetzen des flüssigkeitstragenden Elements (60, 70) in eine Lactatmessvorrichtung (64, 76),
wobei die Lactatmessvorrichtung in der Lage ist, Daten an entfernte Geräte elektronisch zu übertragen, wie etwa durch drahtlose Übertragung, den Fortschritt eines Geburtsvorgangs vorauszusagen.

2. Verfahren nach Anspruch 1, wobei das Verfahren ferner ein Platzieren des Fruchtwassers auf einem flüssigkeitstragenden Element in Form eines Teststreifens (60) umfasst.

3. Verfahren nach Anspruch 2, wobei das Verfahren ferner ein Einsetzen des Teststreifens in ein Einweggehäuse (70) umfasst.

4. Verfahren nach einem der vorhergehenden Ansprüche, wobei das Verfahren ferner ein Markieren des flüssigkeitstragenden Elements und ein Einstellen der Lactatmessvorrichtung (64, 76) zum Detektieren markierter flüssigkeitstragender Elemente umfasst.

5. Verfahren nach Anspruch 1 oder 4, wobei die Lactatmessvorrichtung (64, 76) eine von einem Katheter getragene Flüssigkeit misst.

6. Verfahren nach einem der vorhergehenden Ansprüche, wobei das Verfahren ferner ein Hinzufügen eines Identifizierungsmechanismus auf dem flüssigkeitstragenden Element (60, 70) umfasst.

7. Verfahren nach Anspruch 6, wobei die Lactatmessvorrichtung (64, 76) einen Identifizierungsmechanismus auf dem flüssigkeitstragenden Element (60, 70) vor einem Bestimmen einer Lactatkonzentration des Fruchtwassers (62) detektiert.

8. Verfahren nach einem der vorhergehenden Ansprüche, wobei das Verfahren ferner ein Markieren des flüssigkeitstragenden Elements (60, 70) durch Bereitstellen eines Strichcodes umfasst.

9. Verfahren nach Anspruch 1, wobei das Verfahren ferner ein Hinzufügen von Messkalibrierungsdaten, Produktionsdatum und/oder Produktionschargeninformationen zu dem flüssigkeitstragenden Element (60, 70) umfasst.

10. System zum Entnehmen von Fruchtwasser und Messen von Lactat in diesem *in vitro,* wobei das System Folgendes umfasst:
- eine Lactatmessvorrichtung (64, 76), die in der Lage ist, Daten zu entfernten Geräten elektronisch und drahtlos zu übertragen, und
- ein flüssigkeitstragendes Element (60, 70), das zum Entnehmen einer Fruchtwasserprobe (62), die von einer schwangeren Frau während der Wehen erhalten wird, eingerichtet ist und in die Lactatmessvorrichtung (64, 76) einsetzbar ist, wobei das flüssigkeitstragende Element einen Filter zum Entfernen von Fremdstoffen enthält.

11. System nach Anspruch 10, wobei das flüssigkeitstragende Element markiert ist und einen Identifizierungsmechanismus (66) beinhaltet, der eine Detektion von einem oder mehreren markierten flüssigkeitstragenden Element(en) durch die Messvorrichtung und/oder eine Validierung der Herkunft des flüssigkeitstragenden Elements ermöglicht.

12. System nach Anspruch 11, wobei das markierte flüssigkeitstragende Element Informationen über Produktionsdatum, Kalibrierungsdaten und/oder Produktionscharge trägt.

13. System nach einem der Ansprüche 10-12, wobei die Lactatmessvorrichtung zum Darstellen gemessener Lactatwerte zusammen mit der seit Erhalt des Fruchtwassers verstrichenen Zeit eingerichtet ist, wodurch eine Vorhersage des Fortschritts eines Geburtsvorgangs ermöglicht wird.

14. System nach einem der Ansprüche 10-13, wobei die Lactatmessvorrichtung Alarme basierend auf gemessenen Lactatwerten erzeugen kann.

15. System nach einem der Ansprüche 10-14, wobei die Lactatmessvorrichtung Patienteninformationen zusammen mit gemessenen Lactatwerten speichern kann, wobei die Lactatmessvorrichtung gegebenenfalls Informationen von mehreren Patienten gleichzeitig speichern kann.

16. System nach einem der Ansprüche 10-15, wobei das flüssigkeitstragende Element ein Teststreifen (60) ist.

17. System nach Anspruch 16, wobei der Teststreifen in ein Einweg-Flüssigkeitstragelement (70) eingesetzt worden ist.

18. System nach einem der Ansprüche 10-15, wobei das flüssigkeitstragende Element (60, 70) ein Katheter ist.

19. System nach einem der Ansprüche 10-18, wobei das flüssigkeitstragende Element (60, 70) mit einem Strichcode markiert worden ist.

20. System nach einem der Ansprüche 10-19, wobei die Lactatmessvorrichtung (64, 76) ferner zur Messung von Lactatspiegeln eines Fötus bei oder nach dem Einsetzen der Wehen eingerichtet ist, und wobei das flüssigkeitstragende Element (60, 70) ferner dazu eingerichtet ist, Blut von dem Fötus zu tragen, wobei das Blut gegebenenfalls Kopfhautblut ist.

## Revendications

1. Procédé de surveillance d'un processus d'accouchement d'une femme enceinte, consistant à :
• utiliser un échantillon de liquide amniotique (62) obtenu de la femme enceinte pendant le travail ;
• transporter le liquide amniotique (62) à l'aide d'un élément (60, 70) de transport de liquide ;
• filtrer le liquide amniotique disposé dans l'élément (60, 70) de transport de liquide pour éviter les substances étrangères ; et
• déterminer *in vitro* une concentration en lactate du liquide amniotique (62) transporté par l'élément (60, 70) de transport de liquide en insérant l'élément (60, 70) de transport de liquide dans un dispositif (64, 76) de mesure de lactate,
dans lequel le dispositif de mesure de lactate est capable de transférer les données électroniquement à un équipement distant, tel que par un transfert sans fil,
pour prédire une progression d'un accouchement.

2. Procédé selon la revendication 1, le procédé consistant en outre à placer le liquide amniotique sur un élément de transport de liquide sous la forme d'une bandelette réactive (60).

3. Procédé selon la revendication 2, le procédé consistant en outre à insérer la bandelette réactive dans une enveloppe jetable (70).

4. Procédé selon l'une quelconque des revendications précédentes, le procédé consistant en outre à marquer l'élément de transport de liquide et à régler le dispositif (64, 76) de mesure de lactate pour détecter les éléments de transport de liquide marqués.

5. Procédé selon la revendication 1, ou 4, dans lequel le dispositif (64, 76) de mesure de lactate mesure un liquide transporté par un cathéter.

6. Procédé selon l'une quelconque des revendications précédentes, le procédé consistant en outre à ajouter un mécanisme d'identification sur l'élément (60, 70) de transport de liquide.

7. Procédé selon la revendication 6, dans lequel le dispositif (64, 76) de mesure de lactate détecte un mécanisme d'identification sur l'élément (60, 70) de transport de liquide avant de déterminer une concentration en lactate du liquide amniotique (62).

8. Procédé selon l'une quelconque des revendications précédentes, le procédé consistant en outre à marquer l'élément (60, 70) de transport de liquide en utilisant un code à barres.

9. Procédé selon la revendication 1, le procédé consistant en outre à ajouter des données de mesure d'étalonnage, la date de production, et/ou des informations sur le lot de production sur l'élément (60, 70) de transport de liquide.

10. Système de collecte et de mesure *in vitro* de lactate dans un liquide amniotique, le système comprenant
- un dispositif (64, 76) de mesure de lactate capable de transférer les données, électroniquement et sans fil, à un équipement distant, et
- un élément (60, 70) de transport de liquide, qui est conçu pour collecter un échantillon (62) de liquide amniotique obtenu d'une femme enceinte pendant le travail et qui peut être inséré dans ledit dispositif (64, 76) de mesure de lactate, dans lequel l'élément de transport de liquide contient un filtre permettant d'éliminer les substances étrangères.

11. Système selon la revendication 10, dans lequel l'élément de transport de liquide est marqué et comprend un mécanisme d'identification (66) permettant la détection, par le dispositif de mesure, du ou des éléments de transport de liquide marqués et/ou la validation de l'origine de l'élément de transport de liquide.

12. Système selon la revendication 11, dans lequel l'élément de transport de liquide marqué transporte des informations concernant la date de production, des données d'étalonnage, et/ou le lot de production.

13. Système selon l'une quelconque des revendications 10-12, dans lequel le dispositif de mesure de lactate est conçu pour présenter les valeurs de lactate mesurées conjointement avec le temps écoulé depuis l'obtention du liquide amniotique, permettant une prédiction de progression d'un accouchement.

14. Système selon l'une quelconque des revendications 10-13, dans lequel le dispositif de mesure de lactate peut générer des alarmes basées sur les valeurs de lactate mesurées.

15. Système selon l'une quelconque des revendications 10-14, dans lequel le dispositif de mesure de lactate peut mémoriser les informations concernant le patient conjointement avec les valeurs de lactate mesurées, dans lequel éventuellement le dispositif de mesure de lactate peut mémoriser les informations provenant de multiples patients en même temps.

16. Système selon l'une quelconque des revendications 10-15, dans lequel l'élément de transport de liquide est une bandelette réactive (60).

17. Système selon la revendication 16, dans lequel la bandelette réactive a été insérée dans un élément jetable (70) de transport de liquide.

18. Système selon l'une quelconque des revendications 10-15, dans lequel l'élément (60, 70) de transport de liquide est un cathéter.

19. Système selon l'une quelconque des revendications 10-18, dans lequel l'élément (60, 70) de transport de liquide a été marqué par un code à barres.

20. Système selon l'une quelconque des revendications 10-19, dans lequel le dispositif (64, 76) de mesure de lactate est en outre conçu pour la mesure des taux de lactate d'un foetus au début du travail ou après celui-ci, et dans lequel l'élément (60, 70) de transport de liquide est en outre conçu pour transporter du sang provenant dudit foetus, dans lequel éventuellement le sang est du sang du cuir chevelu.
